(19) Europäisches Patentamt
European Patent Office
Office européen des brevets

(11) Veröffentlichungsnummer: **0 008 645**
**B1**

(12) **EUROPÄISCHE PATENTSCHRIFT**

(45) Veröffentlichungstag der Patentschrift: **05.09.84**

(21) Anmeldenummer: **79102507.5**

(22) Anmeldetag: **18.07.79**

(51) Int. Cl.$^3$: **C 07 D 207/26,**
C 07 D 401/12,
C 07 D 405/12, A 61 K 31/40,
A 61 K 31/495,
A 61 K 31/535, A 61 K 31/435

# (54) Alkoxyphenylpyrrolidone, Verfahren zu ihrer Herstellung und Arzneimittel auf Basis dieser Verbindungen.

(30) Priorität: **01.08.78 DE 2834114**

(43) Veröffentlichungstag der Anmeldung:
**19.03.80 Patentblatt 80/06**

(45) Bekanntmachung des Hinweises auf die Patenterteilung:
**05.09.84 Patentblatt 84/36**

(84) Benannte Vertragsstaaten:
**AT BE CH DE FR GB IT LU NL SE**

(56) Entgegenhaltungen:
**DE-A-2 263 211**
**DE-A-2 337 461**
**DE-A-2 737 630**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Seidelmann, Dieter, Dr.**
**Stierstrasse 14**
**D-1000 Berlin 41 (DE)**
Erfinder: **Schmiechen, Ralph, Dr.**
**Bayernring 27**
**D-1000 Berlin 42 (DE)**
Erfinder: **Paschelke, Gert**
**Hohenzollernstrasse 4**
**D-1000 Berlin 39 (DE)**
Erfinder: **Müller, Bernd, Dr.**
**Sonnenscheinstrasse 30**
**D-5100 Aachen (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 645 B1

**Beschreibung**

Die Erfindung betrifft neue Alkoxyphenylpyrrolidone der allgemeinen Formel I

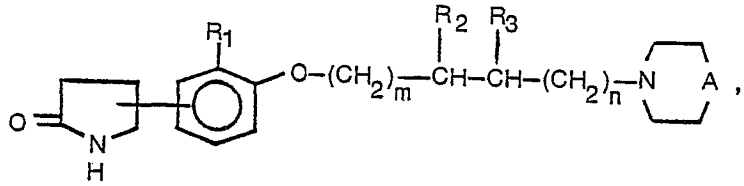

(I)

worin

m und n gleich oder verschieden sind und 0 oder 1,

$R_1 = H$ oder $OCH_3$,

$R_2$, $R_3 = H$, gerad- oder verzweigtkettiges Alkyl mit 1—4 C-Atomen, wobei, wenn $m = 1$ und $n = 0$, $R_2$ auch OH oder aliphatisches Acyloxy mit bis zu 6 C-Atomen, das sich von physiologisch verträglichen Säuren ableitet, oder wenn $m = 0$ und $n = 1$, $R_3$ auch OH oder aliphatisches Acyloxy mit bis zu 6 C-Atomen sein kann, das sich von physiologisch verträglichen Säuren ableitet, und

A = Sauerstoff,

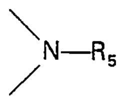 und 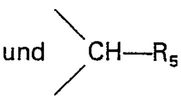

mit $R_5$ in der Bedeutung von H,

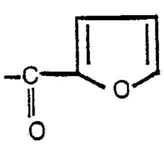, 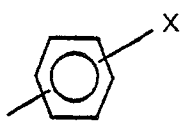, 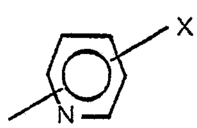, 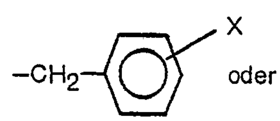 oder 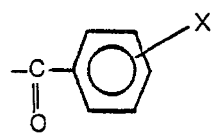

(mit X = H, Alkoxy mit bis zu 4 C-Atomen oder Halogen), bedeuten, und deren Salze.

Unter Alkyl mit 1—4 Kohlenstoffatomen sind beispielsweise Methyl, Äthyl, Propyl, i-Propyl, Butyl, i-Butyl und tert.-Butyl zu verstehen.

Von den Acyloxy gruppen mit bis zu 6 Kohlenstoffatomen, die sich von physiologisch vertraglichen Säuren ableiten, sind solche bevorzugt, die sich von Ameisensäure, Essigsäure, Propionsäure Buttersäure und Capronsäure ableiten.

Die erfindungsgemäßen Verbindungen zeigen eine gefäßerweiternde und blutdrucksenkende Wirkung. Sie zeigen überraschenderweise im Vergleich zu bekannten Antihypertonica eine überlegene Wirksamkeit.

Aus der DE—B—2 263 211 sind bereits Phenoxyalkylamino-Derivate bekannt, bei denen die Pyrrolidon-Gruppe über das N-Atom an die Phenylgruppe gebunden ist, die bei geringer Toxizität blutdrucksenkende Eigenschaften aufweisen.

Die Erfindung betrifft des weiteren ein Verfahren zur Herstellung von Verbindungen der allgemeinen Formel I durch Reaktion von substituierten Phenyl-2-pyrrolidonen der allgemeinen Formel II

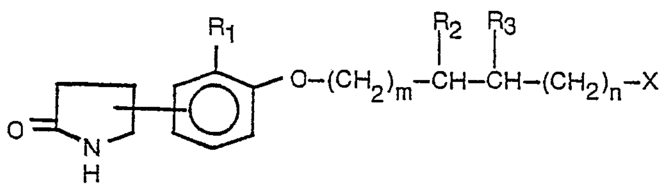

(II),

worin $R_1$, $R_2$, $R_3$, m und n die oben angegebene Bedeutung haben und X Chlor oder mit dem $\beta$-ständigen Kohlenstoffatom eine Epoxidgruppierung darstellt, mit einem sekundäuren cyclischen Amin der allgemeinen Formel III

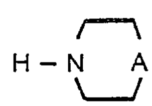

(III),

worin A die oben angegebene Bedeutung besitzt, in an sich bekannter Weise in einem inerten Lösungsmittel bei Temperaturen oberhalb Raumtemperatur und durch eine eventuelle Veresterung freier

Hydroxygruppen und durch eine eventuelle Überführung des so erhaltenen freien Amins der Formel I in das entsprechende Salz.

Das erfindungsgemäße Verfahren der Mischung von Verbindungen der allgemeinen Formel II mit Verbindungen der Formel III enfolgt nach an sich bekannten Methoden.

Geht man von Verbindungen der Formel II aus, bei denen X Chlor bedeutet, wird das sekundäre Amin der Formel III in geringem Überschuß in Gegenwart eines basischen Mittels in einem inerten Lösungsmittel angewendet.

Inerte Lösungsmittel sind beispielsweise aliphatische und aromatische Kohlenwasserstoffe wie Ligroin, Hexan, Benzol und Toluol, Äther wie Diäthyläther, Glykoldimethyläther, Tetrahydrofuran und Dioxan, Alkohol wie Methanol und i-Propanol sowie halogenierte Kohlenwasserstoffe wie Chloroform und Methylenchlorid, sowie Dimethylformamid, Dimethylsulfoxid und Hexamethylphosphortriamid. Diese Lösungsmittel können auch in Gemischen untereinander verwendet werden.

Geeignete basische Mittel sind beispielsweise Alkali- und Erdalkalimetallhydroxide wie Natrium-, Kalium- und Calciumhydroxid, Alkali- und Erdalkalimetallcarbonate wie Kalium- und Magnesiumcarbonat, Alkalimetallalkoholate wie Natriummethylat und Kalium-tert.-butylat und tertiäre Amine wie Triäthylamin und Tributylamin.

Die Reaktions temperatur liegt oberhalb Raumtemperatur. Sie liegt im allgemeinen zwischen 60 und 100°C.

Die Reaktionszeit liegt zwischen einer und acht Stunden.

Geht man von Verbindungen der Formel II aus, bei denen X zusammen mit dem $\beta$-ständigen Kohlenstoffatom eine Epoxidgruppierung bildet, wird das sekundäre Amin zweckmäßigerweise nur in molaren Mengen eingesetzt. Das basische Mittel ist hierbei entbehrlich. Die Reaktion wird in einem inerten Lösungsmittel oder Lösungsmittelgemisch durchgeführt. Reaktionsdauer und -temperatur sind mit derjenigen vergleichbar, die für die entsprechende Reaktion mit Verbindungen der Formel II, in denen X = Cl, angegeben wirde.

Die Ausgangsverbindungen der allgemeinen Formel II, bei denen X zusammen mit dem $\beta$-ständigen Kohlenstoffatom eine Epoxidgruppierung darstellt, werden, soweit sie nicht bekannt sind, aus den entsprechenden Phenolen in Gegenwart eines basischen Kondensationsmittels mit Epichlorhydrin erhalten.

Die Ausgangsverbindungen der allgemeinen Formel II, bei denen X Chlor bedeutet, weden, soweit sie nicht bekannt sind, aus den vorgenannten Epoxiden durch Umsetzung mit Chlorwasserstoffsäure erhalten.

Sie können aber auch direkt durch Umsetzung der entsprechenden Phenole mit Epichlorhydrin in Gegenwart von katalytischen Mengen einer Base, wie z.B. Piperidin, erhalten werden.

Die sich gegebenenfalls anschließende Veresterung einer freien Hydroxygruppe erfolgt gleichfalls nach an sich bekannten Methoden. Eine bevorzugte Methode ist die Reaktion mit einem reaktionsfähigen Säurederivat in Gegenwart eines basischen Katalysators wie z.B. der Umsatz mit einem Säurechlorid oder Säureanhydrid in Gegenwart von Pyridin.

Die Salzbildung des freien Amin erfolgt ebenfalls nach an sich bekannten Methoden. Hierzu wird das Amin mit der gewünschten Säure aus verdünnter Lösung gefällt. Bevorzugt sind Salze mit der Chlorwasserstoffsäure, wobei sich die Hydrochloride bilden.

Die Erfindung betrifft des weiteren Arzneimittel auf Basis der erfindungsgemäßen Verbindungen der Formel I, beispielsweise für die Behandlung von Herzgefäßerkrankungen und des Bluthochdrucks beim Menschen.

Für therapeutische Zwecke werden die erfindungsgemäßen Verbindungen der Formel I oral in einer täglichen Dosis von 0,1 bis 500 mg, vorzugsweise von 1 bis 50 mg, verabreicht.

Die oral zu verabreichenden Darreichungsformen enthalten den Wirkstoff in einer Menge von 0,05 bis 500 mg, vorzugsweise von 0,1 bis 25 mg pro Dosiseinheit.

Für die Verabreichung des Arzneimittels können die Wirkstoffe in Form von Tabletten, eines Granulats, eines Pulvers, von Kapseln u.ä. vorliegen. Der Wirkstoff wird in der Regel zusammen mit Hilfsstoffen oder Trägern verabreicht, wie beispielsweise Lactose, Magnesiumstearat, Kaolin, Saccharose, Maisstärke, Talk, Stearinsäure, Gelatine, Agar-Agar, Pektin usw.

Die nachfolgenden Beispiele sollen die Erfindung erläutern.

Die als Rohprodukte bezeichneten Substanzen wurden durch Dünnschichtchromatographie in mindestens 2 Systemen und mit Hilfe von IR-Spektren auf ausreichende Reinheit geprüft. Alle anderen Substanzen sind analysenrein (C,H,N-Bestimmung, IR-, UV- und NMR-Spektren, Dünnschichtchromatographie).

Die Temperaturen werden in Grad Celsius (°C) angegeben.

Die Schmelzpunkte wurden auf der Koflebank bestimmt.

Die zur Umkristallisation benutzten Lösungsmittel sind in Klammern ( ) hinter den Schmelzpunkten angegeben.

### Beispiel 1

15,5 mMol 4-[3-(2.3-Epoxypropoxy)-4-methoxyphenyl]-2-pyrrolidon werden in 50 ml Methanol gelöst. Nach Zugabe von 15,5 mMol 1-Phenylpiperazin (95%ig) erhitzt man 3 Stunden unter Rückfluß.

Nach dem Erkalten der Reaktionslösung wird das Lösungsmittel im Vakuum bei 40°C abgezogen. Der Rückstand wird in 50 ml 1N Salzsäure aufgenommen und 2mal mit 100 ml Chloroform extrahiert. Die wässrige Phase wird mit 2N Natronlauge alkalisch eingestellt und anschliessend 3mal mit Essigester extrahiert. Die vereinigten Essigesterphasen werden mit gesättigter Natriumchlorid-Lösung gewaschen und das Lösungsmittel nach Trocknen über Natriumsulfat im Vakuum abdestilliert. Der Rückstand wird aus Äthanol umkristallisiert. Man erhält in 61%iger Ausbeute 4-[4-Methoxy-3-(4-phenylpiperazin-1-yl)-2-hydroxypropoxy)-phenyl]-2-pyrrolidon vom Schmelzpunkt 143—144°C.

Aus ätherischer Salzsäure fällt das Dihydrochlorid. Schmelzpunkt 167—169°C.

Das als Ausgangsmaterial benötigte 4-[3-(2.3-Epoxypropoxy)-4-methoxyphenyl]-2-pyrrolidon (F. 124—126°) erhält man durch Umsetzen von 4-(3-Hydroxy-4-methoxyphenyl)-2-pyrrolidon mit Epichlorhydrin und Natriumhydrid in Dimethylformamid.

## Beispiel 2

Nach dem im Beispiel 1 angegebenen Verfahren werden aus 4-[3-(2.3-Epoxypropoxy)-4-methoxyphenyl]-2-pyrrolidon und einem sekundären Amin die in der folgenden Tabelle angegebenen Verbindungen hergestellt, wobei

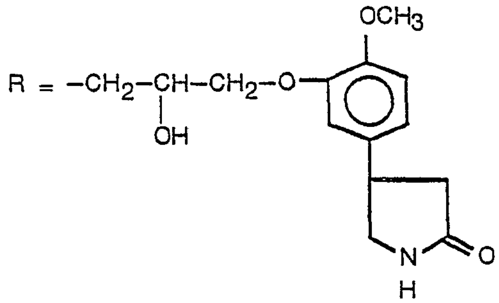

bedeutet.

| Verbindung | Ausbeute % | F. (°C) | Umkristallisiert aus | Temperatur [°C] | Zeit [h] | Lösungs-mittel |
|---|---|---|---|---|---|---|
| 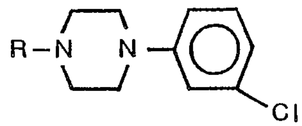 | 63 | 124–126 | Essigester | 65 | 2 | Methanol |
| 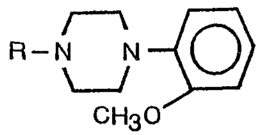 | 54,5 | Öl | Chromatographie MeOH/Chlf.: 2/8 | 65 | 2,5 | Methanol |
| 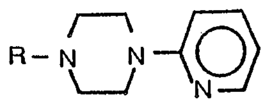 | 61 | 104–106 | Essigester | 65 | 2 | Methanol |
| 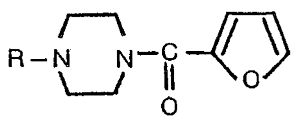 | 33 | Öl | Chromatogr. $CHCl_3$:$CH_3OH$ 9 : 1 | 83 | 4 | i-Propano |
| 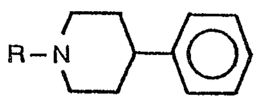 | 64 | 146–147 | Essigester | 65 | 1 | Methanol |
| 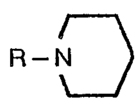 | 20 | 107–108 | Aceton | 78 | 3 | Äthanol |

| Verbindung | Ausbeute %. | F. (°C) | Umkristallisiert aus | Temperatur [°C] | Zeit [h] | Lösungs-mittel |
|---|---|---|---|---|---|---|
| 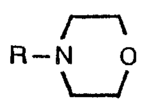 | 83 | 92–94 | Essigester | 65 | 1,5 | Methanol |
| 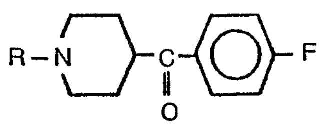 | 47 | 160 | Methanol | 65 | 4 | Methanol |

Beispiel 3

Aus 11,5 mMol 4-[4-(2.3-Epoxypropoxy)-3-methoxyphenyl]-2-pyrrolidon und 11,5 mMol 1-Phenylpiperazin (95%ig) erhält man analog dem Verfahren gemäß Beispiel 1 in 74%iger Ausbeute 4-[3-Methoxy-4-(3-(4-phenylpiperazinyl)-2-hydroxypropoxy)-phenyl]-2-pyrrolidon vom Schmelzpunkt 123—125°C (Äthanol; nach Chromatographie in Methanol/Chloroform: 1/1).

Das als Ausgangsmaterial benötigte 4-[4-(2.3-Epoxypropoxy)-3-methoxyphenyl]-2-pyrrolidon (F. 108—110°) erhält man durch Umsetzen von 4-(4-Hydroxy-3-methoxyphenyl)-2-pyrrolidon mit Epichlorhydrin und Natriumhydrid in Dimethylformamid.

Beispiel 4

Aus 8,6 mMol 4-[3-(2.3-Epoxypropoxy)]-2-pyrrolidon und 8,6 mMol 1-Phenylpiperazin (95%ig) erhält man gemäß Beispiel 1 in 77%iger Ausbeute 4-{3-[2-Hydroxy-3-(4-phenylpiperazinyl)-propoxy]-phenyl]-2-pyrrolidon vom Schmelzpunkt 82—84°C (Äther; nach Chromatographie mit Methanol/CHloroform: 2/8).

Das als Ausgangsmaterial benötigte 4-[3-(2.3-Epoxypropoxy)]-2-pyrrolidon (Öl) erhält man durch Umsetzen von 4-(3-Hydroxyphenyl)-2-pyrrolidon mit Epichlorhydrin und Natriumhydrid in Dimethylformamid.

Beispiel 5

Aus 10 mMol 5-[3-(2.3-Epoxypropoxy)-4-methoxyphenyl]-2-pyrrolidon und 10 mMol 1-Phenylpiperazin (95%ig) erhält man in 52%iger Ausbeute 5-{3-[2-Hydroxy-3-(4-phenylpiperazin-1-yl)-propoxy]-4-methoxyphenyl}-2-pyrrolidon vom Schmelzpunkt 127—131°C (Äthanol).

Das als Ausgangsmaterial benötigte 5-[3-(2.3-Epoxypropoxy)-4-methoxyphenyl]-2-pyrrolidon (F. 140—142°C) erhält man durch Umsetzen von 5-(3-Hydroxy-4-methoxyphenyl)-2-pyrrolidon mit Epichlorhydrin und Natriumhydrid in Dimethylformamid.

Beispiel 6

Aus 10 mMol 3-[3-(2.3-Epoxypropoxy)-4-methoxyphenyl]-2-pyrrolidon und 10 mMol 1-Phenylpiperazin (95%ig) erhält man in 60%iger Ausbeute 3-{3-[2-Hydroxy-3-(4-phenylpiperazin-1-yl)-propoxy]-4-methoxyphenyl}-2-pyrrolidon vom Schemlzpunkt 146—147°C.

Das als Ausgangsmaterial benötigte 3-[3-(2.3-Epoxypropoxy)-4-methoxyphenyl]-2-pyrrolidon (F. 110—112°C) erhält man durch Umsetzen von 3-(3-Hydroxy-4-methoxyphenyl)-2-pyrrolidon mit Epichlorhydrin und Natriumhydrid in Dimethylformamid.

Beispiel 7

20 mMol 4-[3-(2-Chloräthoxy)-4-methoxyphenyl]-2-pyrrolidon werden in 50 ml Dimethylformamid gelöst. Nach Zugabe von 22 mMol 1-Phenylpiperazin (95%ig) und 20 mMol Triäthylamin erhitzt man 6 Stunden auf 100°C. Nach beendeter Umsetzung wird das Lösungsmittel bei 40°C im Hochvakuum abgezogen. Der Rückstand wird in 50 ml Essigester aufgenommen, mit halbgesättigter Natriumchloridlösung gewaschen und das Lösungsmittel nach Trocknen über Natriumsulfat im Vakuum abdestilliert. Man erhält in 15,2%iger Ausbeute 4-{4-Methoxy-3-[2-(4-phenylpiperazin-1-yl)-äthoxy]-phenyl}-2-pyrrolidon vom Schmelzpunkt 140—141°C.

Aus ätherischer Salzsäure fällt das Dihydrochlorid vom Schmelzpunkt 219—221°C.

Das als Ausgangsmaterial benötigte 4-[3-(2-Chloräthoxy)-4-methoxyphenyl]-2-pyrrolidon (F. 134—138°C) erhält man durch Umsetzen von 1-Brom-2-chlor-äthan mit 4-(3-Hydroxy-4-methoxyphenyl)-2-pyrrolidon und Natriumhydrid in Dimethylformamid.

Beispiel 8

Aus 5 mMol 4-[3-(3-Chlorpropoxy)-4-methoxyphenyl]-2-pyrrolidon und 5,5 mMol 1-Phenylpiperazin (95%ig) erhält man gemäß Beispiel 7 in 22,5%iger Ausbeute 4-{4-Methoxy-3-[3-(4-phenylpiperazin-1-yl)-propoxy]-phenyl}-2-pyrrolidon vom Schmelzpunkt 135—137°C (Äthanol).

Das als Ausgangsmaterial benötigte 4-[3-(3-Chlorpropoxy)-4-methoxyphenyl]-2-pyrrolidon (F. 128—130°C) erhält man durch Umsetzen von 4-(3-Hydroxy-4-methoxyphenyl)-2-pyrrolidon mit 1-Brom-3-chlor-propan und Natriumhydrid in Dimethylformamid.

Beispiel 9

Aus 10 mMol 4-[3-(3-Chlorpropoxy)-4-methoxyphenyl]-2-pyrrolidon und 11 mMol 4-(4-Fluorbenzoyl)-piperidin erhält man gemäß Beispiel 7 in 23%iger Ausbeute 4-(4-Methoxy-3-[3-(4-(4-fluorbenzoyl)-piperidin-1-yl)-propoxy]-phenyl}-2-pyrrolidon vom Schmelzpunkt 104—105°C (Äthanol; nach Chromatographie Methanol/Chloroform 9/1).

Beispiel 10

Aus 20 mMol 4-[3-(4-Brombutoxy)-4-methoxyphenyl]-2-pyrrolidon und 22 mMol 1-Plhenylpiperazin (95%ig) erhält man in 30%iger Ausbeute 4-{4-Methoxy-3-[4-(4-phenylpiperazin-1-yl)-butoxy]-phenyl}-2-pyrrolidon vom Schmelzpunkt 123—124°C (Essigester).

Das als Ausgangsmaterial benötigte 4-[3-(4-Brombutoxy)-4-methoxyphenyl]-2-pyrrolidon (F. 116—119°C) erhält man durch Umsetzen von 4-(3-Hydroxy-4-methoxyphenyl)-2-pyrrolidon mit 1.4-Dibrombutan und Natriumhydrid in Dimethylformamid.

Beispiel 11

7,5 mMol 4-[4-Methoxy-3-(3-(piperazin-1-yl)-2-hydroxypropoxyy)-phenyl]-2-pyrrolidon werden in 50 ml trockenem Pyridin gelöst. Nach Zugabe von 15 mMol Acetanhydrid erhitzt man 3 Stunden unter Rückfluß. Nach dem Abkühlen wird das Reaktionsgemisch im Vakuum eingeengt. Der Rückstand wird über Kieselgel chromatographiert (Aceton/Dichlormethan: 1/1) und aus i-Propanol umkristallisiert. Man erhält in 86%iger Ausbeute 4-[4-Methoxy-3-(3-(piperazin-1-yl)-2-acetoxypropoxy)-phenyl]-2-pyrrolidon vom Schmelzpunkt 128—133°C.

Beispiel 12

Nach dem im Beispiel 1 angegebenen Verfahren werden aus 10 mMol 4-[3-(1-Methyl-2.3-epoxy)-4-methoxyphenyl]-2-pyrrolidon und 10 mMol 1-Phenylpiperazin (95%ig) in 68%iger Ausbeute 4-[4-Methoxy-3-(3-(4-piperazin-1-yl)-1-methyl-2-hydroxypropoxy)-phenyl]-2-pyrrolidon vom Schmelzpunkt 153—155°C (Äthanol) erhalten.

Das als Ausgangsmaterial benötigte 4-[3-(1-Methyl-2.3-epoxy)-4-methoxyphenyl]-2-pyrrolidon (F. 121—125°C) erhält man durch Umsetzen von 4-(3-Hydroxy-4-methoxyphenyl)-2-pyrrolidon mit 3-Brom-1.2-epoxy-butan (hergestellt nach M. Santelli THL 1977(50), Seite 4397) und Natriumhydrid in Dimethylformamid.

Beispiel 13

Aus 10 mMol 4-[3-(3-Brombutoxy)-4-methoxyphenyl]-2-pyrrolidon und 11 mMol 1-Phenylpiperazin (95%ig) erhält man analog Beispiel 7 in 19,8%iger Ausbeute 4-{4-Methoxy-3-[3-(3-methyl-3-(4-phenylpiperazin-1-yl)-propoxy]-phenyl}-2-pyrrolidon vom Schmelzpunkt 88—90°C (Äthanol; nach Chromatographie mit Methanol/Chloroform 1/9).

Aus ätherischer Salzsäure wird das Dihydrochlorid vom Schmelzpunkt 120°C (Zersetzung) gefällt.

Das als Ausgangsmaterial verwendete 4-[3-(3-Brombutoxy)-4-methoxyphenyl]-2-pyrrolidon (öl) erhält man durch Umsetzen von 1.3-Dibrombutan mit 4-(3-Hydroxy-4-methoxyphenyl)-2-pyrrolidon und Natriumhydrid in Dimethylformamid.

**Patentansprüche**

1. Alkoxyphenylpyrrolidone der allgemeinen Formel I

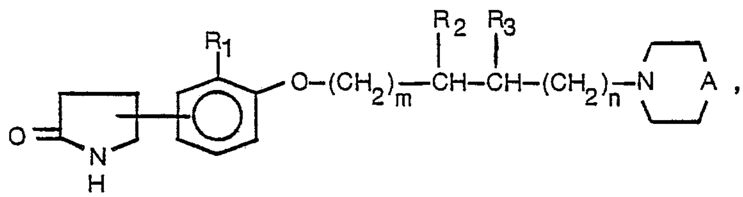

(I)

worin

m und n gleich oder verschieden sind und 0 oder 1,

$R_1 = H$ oder $OCH_3$,

$R_2$, $R_3 = H$, gerad- oder verzweigtkettiges Alkyl mit 1—4 C-Atomen, wobei, wenn $m = 1$ und $n = 0$, $R_2$ auch OH oder aliphatisches Acyloxy mit bis zu 6 C-Atomen, das sich von physiologisch verträglichen Säuren ableitet, oder wenn $m = 0$ und $n = 1$, $R_3$ auch OH oder aliphatisches Acyloxy mit bis zu 6 C-Atomen sein, das sich von physiologisch verträglichen Säuren ableitet, kann und

A = Sauerstoff.

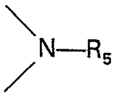 und 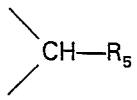

mit $R_5$ in der Bedeutung von H,

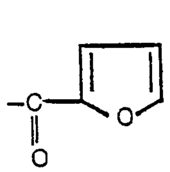, 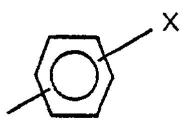, 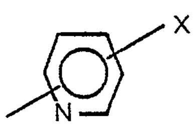, 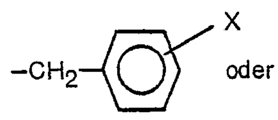 oder 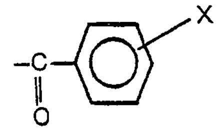

(mit X = H, Alkoxy mit bis zu 4 C-Atomen oder Halogen), bedeuten, und deren Salze.

2. 4-{4-Methoxy-3-[3-(4-phenylpiperazin-1-yl)-2-hydroxypropoxy]-phenyl}-2-pyrrolidon.
3. 4-{4-Methoxy-3-[3-(4-(3-chlorphenyl)-piperazin-1-yl)-2-hydroxypropoxy]-phenyl}-2-pyrrolidon.
4. 4-{4-Methoxy-3-[3-(4-(2-methoxyphenyl)-piperazin-1-yl)-2-hydroxypropoxy]-phenyl}-2-pyrrolidon.
5. 4-{4-Methoxy-3-[3-(4-(2-pyridino)-piperazin-1-yl)-2-hydroxypropoxy]-phenyl}-2-pyrrolidon.
6. 4-{4-Methoxy-3-[3-(4-(2-carboxyfuryl)-piperazin-1-yl)-2-hydroxypropoxy]-phenyl}-2-pyrrolidon.
7. 4-{4-Methoxy-3-[3-(piperidin-1-yl)-2-hydroxypropoxy]-phenyl}-2-pyrrolidon.
8. 4-[4-Methoxy-3-(3-morpholinyl)-2-hydroxypropoxy)-phenyl]-2-pyrrolidon.
9. 4-{4-Methoxy-3-[3-(4-(4-fluorbenzoyl)-piperidin-1-yl)-2-hydroxypropoxy]-phenyl}-2-pyrrolidon.
10. 4-[3-Methoxy-4-(3-(4-phenylpiperazin-1-yl)-2-hydroxypropoxy)-phenyl]-2-pyrrolidon.
11. 4-{4-Methoxy-3-[3-(4-phenylpiperidin-1-yl)-2-hydroxypropoxy]-phenyl}-2-pyrrolidon.
12. 4-{3-[2-Hydroxy-3-(4-phenylpiperazin-1-yl)-propoxy]-phenyl}-2-pyrrolidon.
13. 5-{3-[2-Hydroxy-3-(4-phenylpiperazin-1-yl)-propoxy]-4-methoxyphenyl}-2-pyrrolidon.
14. 3-{-[2-Hydroxy-3-(4-phenylpiperazin-1-yl)-propoxy]-4-methoxyphenyl}-2-pyrrolidon.
15. 4-{4-Methoxy-3-[2-(4-phenylpiperazin-1-yl)-äthoxy]-phenyl}-2-pyrrolidon und dessen Dihydrochlorid.
16. 4-{4-Methoxy-3-[3-(4-phenylpiperazin-1-yl)-propoxy]-phenyl}-2-pyrrolidon.
17. 4-{4-Methoxy-3-[3-(4-(4-fluorbenzoyl)-piperidin-1-yl)-propoxy]-phenyl}-2-pyrrolidon.
18. 4-{4-Methoxy-3-[4-(4-phenylpiperazin-1-yl)-butoxy]-phenyl}-2-pyrrolidon.
19. 4-[4-Methoxy-3-(3-(piperazin-1-yl)-2-acetoxypropoxy)-phenyl]-2-pyrrolidon.
20. 4-[4-Methoxy-3-(3-(piperazin-1-yl)-1-methyl-2-hydroxypropoxy)-phenyl]-2-pyrrolidon.
21. 4-{4-Methoxy-3-[3-(piperazin-1-yl)-2-hydroxypropoxy]-phenyl}-2-pyrrolidon-dihydrochlorid.
22. 4-{4-Methoxy-3-[3-(3-methyl-3-(4-phenylpiperazin-1-yl)-propoxy]-phenyl}-2-pyrrolidon.
23. Verfahren zur Herstellung von Verbindungen gemäß Anspruch 1 dadurch gekennzeichnet, daß man substituierte Phenyl-2-pyrrolidone der allgemeinen Formel II

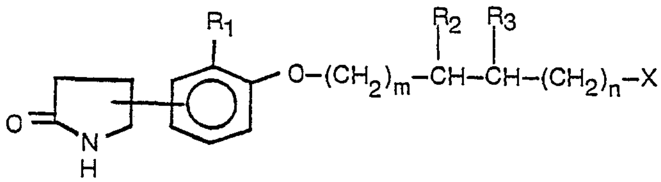 (II),

worin $R_1$, $R_2$, $R_3$, m und n die in Anspruch 1 angegebene Bedeutung haben und X Chlor oder mit dem β-ständigen Kohlenstoffatom eine Epoxidgruppierung darstellt, mit einem sekundären cyclischen Amin der allgemeinen Formel III

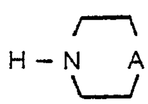 (III),

worin A die in Anspruch 1 angegebene Bedeutung besitzt, in an sich bekannter Weise in einem inerten Lösungsmittel bei Temperaturen oberhalb Raumtemperatur kondensiert und gegebenenfalls vorhandene freie Hydroxygruppen in an sich bekannter Weise verestert und das erhaltene freie Amin der Formel I gemäß Anspruch 1 gegebenenfalls mit einer Säure, vorzugsweise mit Chlorwasserstoffsäure, in sein Salz überführt.
24. Arzneimittel auf Basis von Verbindungen gemäß Anspruch 1—23.

**Claims**

1. Aminoalkoxyphenylpyrrolidones of the general formula I

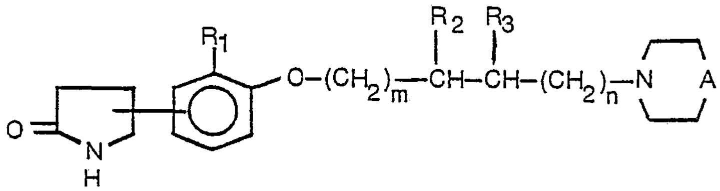 (I),

wherein
m and n are each, independently, integers of 0 and 1;
$R_1$ is H or $OCH_3$;

$R_2$ and $R_3$ are each independently, H, straight or branched chain $C_{1-4}$ alkyl, and if m = 1 and n = 0 $R_2$ is also OH or aliphatic acyloxy with up to 6 C-atoms which is derived from a physiologically acceptable acid, or, if m = 0 and n = 1 $R_3$ is also aliphatic acyloxy with up to 6 C-atoms which is derived from a physiologically acceptable acid; and

A is oxygen,

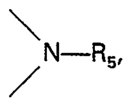 and 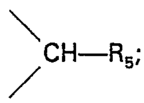

$R_5$ is H,

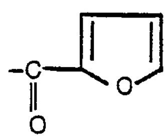 , 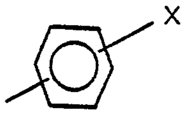 , 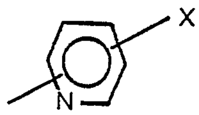 , 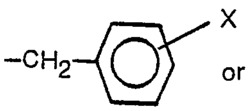 or 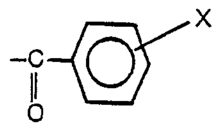

(X is H, $C_{1-4}$ alkoxy or halogen), and salts thereof.

2. 4-{4-Methoxy-3-[3-(4-phenylpiperazin-1-yl)-2-hydroxypropoxy]phenyl}-2-pyrrolidone.

3. 4-{4-Methoxy-3-[3-(4-(3-chlorophenyl)piperazin-1-yl)-2-hydroxypropoxy]phenyl}-2-pyrrolidone.

4. 4-{4-Methoxy-3-[3-(4-(2-methoxyphenyl)piperazin-1-yl)-2-hydroxypropoxy]phenyl}-2-pyrrolidone.

5. 4-{4-Methoxy-3-[3-(4-(2-pyridino)piperazin-1-yl)-2-hydroxypropoxy]phenyl}-2-pyrrolidone.

6. 4-{4-Methoxy-3-[3-(4-(2-carboxyfuryl)piperazin-1-yl)-2-hydroxypropoxy]phenyl}-2-pyrrolidone.

7. 4-{4-Methoxy-(3-piperidin-1-yl)-2-hydroxypropoxy)phenyl}-2-pyrrolidone.

8. 4-{4-Methoxy-3-[3-morpholinyl)-2-hydroxypropoxy)phenyl}-2-pyrrolidone.

9. 4-{4-Methoxy-3-[3-(4-(4-fluorobenzoyl)-piperidin-1-yl)-2-hydroxypropoxy]phenyl}-2-pyrrolidone.

10. 4-{3-Methoxy-4-[3-(4-phenylpiperazin-1-yl)-2-hydroxypropoxy]phenyl}-2-pyrrolidone.

11. 4-{4-Methoxy-3-[3-(4-phenylpiperidin-1-yl)-2-hydroxypropoxy]phenyl}-2-pyrrolidone.

12. 4-{3-[2-Hydroxy-3-(4-phenylpiperazin-1-yl)-propoxy]phenyl}-2-pyrrolidone.

13. 5-{3-[2-Hydroxy-3-(4-phenylpiperazin-1-yl)-propoxy-4-methoxy]phenyl}-2-pyrrolidone.

14. 3-{3-[2-Hydroxy-3-(4-phenylpiperazin-1-yl)propoxy]-4-methoxylphenyl}-2-pyrrolidone.

15. 4-{4-Methoxy-3-[2-(4-phenylpiperazin-1-yl)-ethoxy]phenyl}-2-pyrrolidone and its dihydrochloride.

16. 4-{4-Methoxy-3-[3-(4-phenylpiperazin-1-yl)-propoxy]phenyl}-2-pyrrolidone.

17. 4-{4-Methoxy-3-[3-(4-(4-fluorbenzoyl)-piperidin-1-yl)-propoxy]phenyl}-2-pyrrolidone.

18. 4-{4-Methoxy-3-[4-(4-phenylpiperazin-1-yl)-butoxy]phenyl}-2-pyrrolidone.

19. 4-{4-Methoxy-3-[3-(piperazin-1-yl)-2-acetoxypropoxy]phenyl}-2-pyrrolidone.

20. 4-{4-Methoxy-3-(3-(piperazin-1-yl)-1-methyl-2-hydroxypropoxy)phenyl}-2-pyrrolidone.

21. 4-{4-Methoxy-3-[3-piperazin-1-yl)-2-hydroxypropoxy]phenyl}-2-pyrrolidone-dihydrochloride.

22. 4-{4-Methoxy-3-[3-(3-methyl-3-(4-phenylpiperazin-1-yl)propoxy]phenyl}-2-pyrrolidone.

23. A method for preparing compounds according to claim 1 characterized by condensing substituted phenyl-2-pyrrolidones of the Formula II

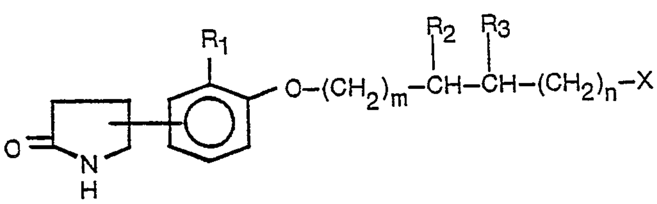 (II),

wherein $R_1$, $R_2$, $R_3$, m and n are as defined for Formula I; and X is chlorine or an epoxy group linked to the carbon atom in the $\beta$-position; with a secondary cyclic amine of the general Formula III

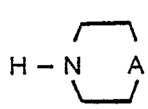 (III),

wherein A is a defined for Formula I; in a manner known per se, in an inert solvent, at temperatures

above room temperature; and optionally esterifying free hydroxy groups by known methods, and, if desired, converting the thus-obtained free amine of Formula I with an acid, preferably with hydrochloric acid, into the corresponding salt.

24. A pharmaceutical composition on the basis of compounds according to claim 1—23.

## Revendications

1. Alcoxyphénylpyrrolidones répondant à la formule générale I

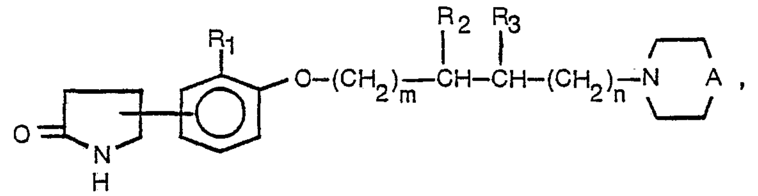 (I),

dans laquelle m et n sont identiques ou différents et ont une valeur de 0 ou de 1, $R_1$ = H ou $OCH_3$, $R_2$, $R_3$ = H, ou un radical alkyle linéaire ou ramifié ayant de 1 à 4 atomes de carbone, $R_2$ pouvant aussi, lorsque m = 1 et n = 0, représenter OH ou un groupe acyloxy aliphatique ayant jusqu'à 6 atomes de carbone, qui derive d'acides physiologiquement capatibles, $R_3$ pouvant aussi, lorsque m = 0 et n = 1, représenter OH ou un groupe acyloxy aliphatique ayant jusqu'à 6 atomes de carbone qui dérive d'acides physiologiquement compatibles, et A = oxygène,

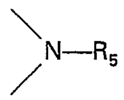 où 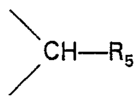

où $R_5$ représente H,

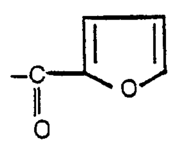 , 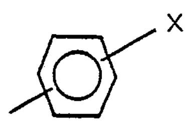 , 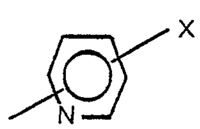 , 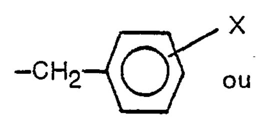 ou 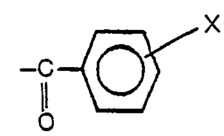

(où X = H, groupe alcoxy ayant jusqu'à 4 atomes de carbone ou halogène), et les sels de ces composés.

2. La 4-{4-méthoxy-3-[3-(4-phénylpipérazin-1-yl)-2-hydroxy-propoxy]-phényl}-2-pyrrolidone.

3. La 4-{4-méthoxy-3-[3-(4-(3-chlorophényl)-pipérazin-1-yl)-2-hydroxypropoxy]-phényl}-2-pyrrolidone.

4. La 4-{4-méthoxy-3-[3-(4-(2-méthoxyphényl)-pipérazin-1-yl)-2-hydroxypropoxy]-phényl}-2-pyrrolidone.

5. La 4-{4-méthoxy-3-[3-(4-(2-pyridino)-pipérazin-1-yl)-2-hydroxypropoxy]-phényl}-2-pyrrolidone.

6. La 4-{4-méthoxy-3-[3-(4-(2-carboxyfuryl)-pipérazin-1-yl)-2-hydroxypropoxy]-phényl}-2-pyrrolidone.

7. La 4-{4-méthoxy-3-[3-(pipéridin-1-yl)-2-hydroxypropoxy]-phényl}-2-pyrrolidone.

8. La 4-[4-méthoxy-3-(3-(morpholinyl)-2-hydroxypropoxy)-phényl]-2-pyrrolidone.

9. La 4-{4-méthoxy-3-[3-(4-(4-fluorobenzoyl)-pipéridin-1-yl)-2-hydroxypropoxy]-phényl}-2-pyrrolidone.

10. La 4-[3-méthoxy-4-(3-(4-phénylpipérazin-1-yl)-2-hydroxypropoxy)-phényl]-2-pyrrolidone.

11. La 4-{4-méthoxy-3-[3-(4-phénylpipéridin-1-yl)-2-hydroxy-propoxy]-phényl}-2-pyrrolidone.

12. La 4-{3-[2-hydroxy-3-(4-phénylpipérazin-1-yl)-propoxy]-phényl}-2-pyrrolidone.

13. La 5-{3-[2-hydroxy-3-(4-phénylpipérazin-1-yl)-propoxy]-4-méthoxyphényl}-2-pyrrolidone.

14. La 3-{3-[2-hydroxy-3-(4-phénylpipérazin-1-yl)-propoxy]-4-méthoxyphényl}-2-pyrrolidone.

15. La 4-{4-méthoxy-3-[2-(4-phénylpipérazin-1-yl)-éthoxy]-phényl}-2-pyrrolidone et son dichlorhydrate.

16. La 4-{4-méthoxy-3-[3-(4-phénylpipérazin-1-yl)-propoxy]-phényl}-2-pyrrolidone.

17. La 4-{4-méthoxy-3-[3-(4-(4-fluorobenzoyl)-pipéridin-1-yl)-propoxy]-phényl}-2-pyrrolidone.

18. La 4-{4-méthoxy-3-[4-(4-phénylpipérazin-1-yl)-butoxy]-phényl}-2-pyrrolidone.

19. La 4-[4-méthoxy-3-(3-(pipérazin-1-yl)-2-acétoxypropoxy)-phényl]-2-pyrrolidone.

20. La 4-[4-méthoxy-3-(3-(pipérazin-1-yl)-1-méthyl-2-hydroxy-propoxy)-phényl]-2-pyrrolidone.

21. Le dichlorhydrate de 4-{4-méthoxy-3-[3-(pipérazin-1-yl)-2-hydroxypropoxy]-phényl}-2-pyrrolidone.

22. La 4-{4-méthoxy-3-[3-(3-méthyl-3-(4-phénylpipérazin-1-yl)-propoxy]-phényl}-2-pyrrolidone.

23. Procédé de préparation de composés suivant la revendication 1, caractérisé en ce qu'on condense des phényl-2-pyrrolidones substituées répondant à la formule générale II

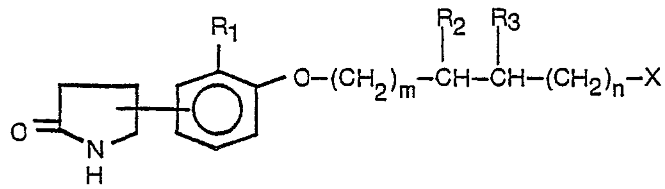

(II),

dans laquelle $R_1$, $R_2$, $R_3$, m et n ont la même signification que celle donnée dans la revendication 1 et X représente du chlore ou un groupement époxy avec l'atome de carbone et position $\beta$, avec une amine cyclique secondaire de la formule générale III

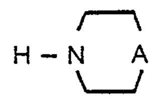

(III),

dans laquelle A a la même signification que celle donnée dans la revendication 1, d'une manière connue en soi dans un solvant inerte à des températures supérieures à la température ambiante, et on estérifie d'une manière connue en soi des groupes hydroxy libres éventuellement présents et on convertit éventuellement l'amine libre obtenue répondant à la formule I suivant la revendication 1 en un sel par un acide, de préférence de l'acide chlorhydrique.

24. Médicaments à base de composés suivant l'une des revendications 1 à 23.